# EUROPÄISCHE PATENTSCHRIFT

(11) **EP 0 713 849 B1**
(45) Veröffentlichungstag und Bekanntmachung des Hinweises auf die Patenterteilung: **11.03.1998**
(21) Anmeldenummer: 95118130.4
(22) Anmeldetag: 17.11.1995
(51) Int. Cl.: C07C 29/60, C07C 31/20

(54) **Verfahren zur Herstellung von Propandiol-1,2**
Process for the preparation of 1,2-propanediol
Procédé pour la préparation de 1,2-propanediol

(30) Priorität: 26.11.1994 DE 4442124
(43) Veröffentlichungstag der Anmeldung: 29.05.1996
(73) Patentinhaber: BASF AKTIENGESELLSCHAFT, 67056 Ludwigshafen (DE)
(72) Erfinder: Schuster, Ludwig, Dr., D-67117 Limburgerhof (DE); Eggersdorfer, Manfred, Dr., D-67227 Frankenthal (DE)

(56) Entgegenhaltungen:
- EP-A- 0 382 050
- EP-A- 0 415 202
- EP-A- 0 523 014
- EP-A- 0 523 015
- DE-A- 4 302 464

## Beschreibung

Die Erfindung betrifft ein Verfahren zur Herstellung von Propandiol-1,2 durch die katalytische Hydrierung von Glycerin bei erhöhter Temperatur und erhöhtem Druck, bei dem man Glycerin mit einem Wassergehalt von bis zu 20 Gew.-% einsetzt und einen Katalysator verwendet, der die Metalle Cobalt, Kupfer, Mangan und Molybdän in Mengen von, bezogen auf die Gesamtmenge des Katalysators,
- 40 bis 70 Gew.-%: Cobalt
- 10 bis 20 Gew.-%: Kupfer
- 0 bis 10 Gew.-%: Mangan und
- 0 bis 10 Gew.-%: Molybdän
enthält, wobei dieser katalytisch aktiven Masse außerdem noch anorganische Polysäuren und/oder Heteropolysäuren in einer Menge von bis zu 10 Gew.-%, bezogen auf die Gesamtmasse des Katalysators, zugesetzt sein können.

Die Hydrierung von Glycerin zu Propandiol-1,2 wurde bereits häufig untersucht, wobei jedoch die ungenügende Selektivität die Anwendung in der Produktion verhinderte. In zwei jüngeren Patentanmeldungen wird die Hydrierung von Glycerin an modifizierten Rutheniumträgerkatalysatoren (EP-A-0 523 014) und an Zink-Kupfer-Katalysatoren (EP-A-0 523 015) beschrieben. Wenn darin auch gewisse Fortschritte gegenüber den früheren Arbeiten erzielt worden sind, haben diese Arbeitsweisen doch noch einige Nachteile.

So wird beispielsweise in beiden Anmeldungen die Hydrogenolysereaktion in sehr verdünnten wäßrigen Lösungen ausgeführt. Die überwiegend verwendeten 30%igen Lösungen von Glycerin in Wasser verdünnen sich durch das entstehende Reaktionswasser noch weiter. Zur Gewinnung von Propandiol muß daher eine große Menge Wasser vorher abdestilliert werden, was einen hohen Energieaufwand bedeutet.

Ferner läßt die Selektivität noch zu wünschen übrig. Wenn ein fast quantitativer Umsatz erzielt wird, geht dies auf Kosten der Selektivität. Man erhält dann in der Regel bei diskontinuierlicher Fahrweise nur Selektivitätswerte zwischen 75 und 84 %.

Um 100%igen Umsatz zu erzielen, waren beim Ruthenium-Katalysator der EP-A-0 523 014 außerdem 10 bis 45 Gew.-% Natriumhydroxid, bezogen auf Glycerin, notwendig.

In der EP-A-0 415 202 ist ein Verfahren zur Herstellung niederer, mehrwertiger Alkohole durch die katalytische Hydrierung wäßriger Saccharoselösungen bei erhöhter Temperatur und bei erhöhtem Druck und mittels eines Katalysators, dessen aktive Masse im wesentlichen die Metalle Cobalt, Kupfer und Mangan enthält, beschrieben.

Aufgabe der vorliegenden Erfindung war es, ein Verfahren zur Herstellung von Propandiol-1,2 durch die katalytische Hydrierung von Glycerin bei erhöhter Temperatur und erhöhtem Druck bereitzustellen, welches die vorstehend geschilderten Nachteile nicht aufweist.

Demgemäß wurde das eingangs definierte Verfahren gefunden.

Der im erfindungsgemäßen Verfahren eingesetzte Katalysator ist im allgemeinen im wesentlichen frei von Katalysatorträgern und enthält vorzugsweise neben Cobalt und Kupfer noch Mangan und Molybdän sowie Phosphorsäure und Phosphat.

In einer bevorzugten Ausführungsform enthält der Katalysator 40 bis 70 Gew.-% Cobalt (als CoO), 13 bis 17 Gew.-% Kupfer (als CuO) und 3 bis 8 Gew.-% Mangan (als MnO₂) sowie 0,1 bis 5 Gew.-% Phosphor (als H₃PO₄) und 0,5 bis 5 Gew.-% Molybdän (als MoO₃), jeweils bezogen auf die Gesamtmasse des Katalysators.

Besonders bevorzugt sind solche Katalysatoren, welche 55 bis 70 Gew-% CoO, 13 bis 17 Gew.-% CuO, 4 bis 6 Gew.-% MnO₂, 1 bis 3 Gew.-% Phosphor (als Phosphorsäure) und 1 bis 4 Gew.-% MoO₃ enthalten.

Die im erfindungsgemäßen Verfahren verwendeten Katalysatoren werden im allgemeinen so hergestellt, daß man diese Metalle, beispielsweise als Oxide, Hydroxide, Oxidhydrate, basische Salze, oder Carbonate aus einer Lösung ihrer Salze, vorzugsweise ihrer Nitrate oder Acetate, durch Zugabe einer Base, vorteilhaft einer wäßrigen Mineralbase wie Natronlauge, Kalilauge, oder Natriumcarbonatlösung gemeinsam fällt und den Niederschlag abtrennt, trocknet und calciniert. Vorzugsweise wird die Fällung nach dem Verfahren der DE-A-23 21 101 durchgeführt.

Vor seiner Verwendung im erfindungsgemäßen Verfahren wird der auf diese Weise erhaltene Katalysator durch Reduktion mit Wasserstoff aktiviert, wobei die darin enthaltenen Metallverbindungen ganz oder teilweise zu den entsprechenden Metallen reduziert werden. Im allgemeinen wird diese Reduktion bei erhöhter Temperatur, vorzugsweise bei Temperaturen von 200 bis 400 °C, im Wasserstoffstrom vorgenommen.

Katalysatoren mit besonders vorteilhaften mechanischen Eigenschaften können erhalten werden, indem man der Metallsalzlösung vor der Fällung anorganische Säuren, die zur Bildung von Polysäuren oder Heteropolysäuren befähigt sind, wie Schwefelsäure, Borsäure, Phosphorsäure, Molybdänsäure, Vanadinsäure, Wolframsäure und/oder deren Salze wie Trinatriumphosphat, Natriumtetraborat, Kaliumdihydrogenphosphat, Calciumhydrogenphosphat, Magnesiumhydrogenborat, Aluminiumphosphat, Natriummolybdat, Ammoniummolybdat, Ammoniumvanadat und/oder Natriumwolframat zusetzt und dann die Fällung und weitere Behandlung des Niederschlags wie in der EP-A-0 415 202 beschrieben durchführt.

Die Zusammensetzung der mit den beschriebenen Katalysatoren erhältlichen Hydrierprodukte hängt von der gewählten Reaktionstemperatur ab. So lassen sich besonders hohe Ausbeuten an Propandiol-1,2 erzielen, wenn die erfindungsgemäße Hydrierung von Glycerin bei Temperaturen von 180 bis 270 °C, vorzugsweise 200 bis 250 °C, durchgeführt wird.

Die Hydrierung von Glycerin wird erfindungsgemäß im allgemeinen mit Wasserstoffdrücken von 100 bis 700 bar, vorzugsweise bei Drücken von 200 bis 325 bar, ausgeführt. Bei niedrigeren Drücken wird die Reaktion zu langsam und unvollständig, bei Drücken über 700 bar werden in immer größerem Maße einwertige Alkohole gebildet. Es kann sogar zu einer zunehmenden Kettenspaltung kommen.

Im erfindungsgemäßen Verfahren wird bevorzugt Glycerin sehr hoher Konzentration, beispielsweise vom Monohydrat bis zum reinen Glycerin, hydriert. Die Hydrierung schwächer konzentrierter Glycerinlösungen mit einem Wassergehalt von bis zu 20 Gew.-% ist im Hinblick auf die Zusammensetzung des Hydrierproduktes ebenfalls mit gutem Erfolg möglich, allerdings auf Grund der verringerten Raum-Zeit-Ausbeute wenig wirtschaftlich. Dies gilt auch für die energetisch aufwendigere Aufkonzentrierung der wäßrigen Lösungen.

Es ist erfindungsgemäß möglich, auch nichtwäßrige Lösungen, beispielsweise in Methanol, einzusetzen; dies bringt jedoch keinen wesentlichen Vorteil gegenüber dem konzentrierten Glycerin.

Wenn im erfindungsgemäßen Verfahren Glycerin aus der Umesterung von Fetten und Ölen eingesetzt wird, sollte dies zweckmäßigerweise vor der Hydrierung von Katalysatorgiften wie Schwefel befreit werden. Dies gelingt beispielsweise durch einfache Kurzwegdestillation. Bei Rohglycerin, welches die als Umesterungskatalysator oft verwendete Schwefelsäure enthält, kann eine Behandlung mit Ionenaustauschern schon ausreichen.

Die Erfindung hat zahlreiche Vorteile. So kann man hochprozentiges Glycerin, beispielsweise das Monohydrat mit 86 Gew.% Glycerin, oder sogar reines Glycerin, mit Ausbeuten von im allgemeinen bis zu 95 % selektiv zu Propandiol-1,2 hydrieren.

Die erfindungsgemäße Hydrierung gibt daneben sowohl in Autoklaven als auch in einem mit körnigem Katalysator gefüllten Rieselreaktor bei vollständigem Umsatz Selektivitäten bis 95 %. In geringem Maße entstehen daneben Methanol, Ethanol, Isopropanol und Propandiol-1,3.

Die Prozentangaben in den Beispielen beziehen sich auf das Gewicht.

### Beispiele

### Beispiel 1

In einem Autoklav von 1,2 l Inhalt, der mit einem schnellaufenden Begasungsrührer versehen war, wurden 700 g 99,5%iges Glycerin und 50 g eines Katalysators aus 68 Gew.-% Cobalt (als CoO), 17 Gew.-% Kupfer (als CuO) und 6 Gew.-% Mangan (als MnO₂) sowie 4 Gew.-% Phosphor (als H₃PO₄) und 5 Gew.-% Molybdän (als MoO₃) vorgelegt. Dieser war vorher bei 300°C mit Wasserstoff reduziert worden.

Dann wurden in kaltem Zustand Wasserstoff bis zu einem Druck von 50 bar aufgepreßt und hierauf der Autoklav auf 250 °C hochgeheizt. Nach dem Erreichen dieser Reaktionstemperatur wurde der Druck auf 250 bar erhöht und durch Nachpressen auf dieser Höhe gehalten. Nach 6 Stunden wurde der Autoklav abgekühlt, entleert und der Inhalt vom Katalysator abfiltriert. Es wurden 687 g Reaktionsmischung mit einem Wassergehalt von 23,6 % erhalten.

Die Analyse erfolgte durch HPLC mit einer Aminex HPX 87C-Säule und einem Brechungs-Index-Detector. Es wurden 95,8 Gew.-% Propandiol-1,2 und 3,2 Gew.-% n-Propanol gefunden. Glycerin war nicht mehr nachweisbar.

### Beispiel 2

Als Reaktor für die kontinuierliche Ausgestaltung des erfindungsgemäßen Verfahrens diente ein Rieselturm von 10 m Länge und einer lichten Weite von 4,5 cm. Die Hydriersäule wurde mit 21,9 kg des unter Beispiel 1 beschriebenen Katalysators gefüllt. Durch langsames Erhitzen auf 300 °C, zuerst in einem H₂/N₂-Gemisch mit 10 % Wasserstoff, dann ansteigend bis zum reinen Wasserstoff wurde der Katalysator in den metallischen Zustand übergeführt.

Die Reaktionsbedingungen waren wie folgt:
Zulauf: 86,5%iges Glycerin (Rest Wasser)
Druck: 295 bar
Temperatur: 210 bis 220 °C
Flüssigkeitsumlauf: 35 l/h = 22 m³/m²h
Gasumlauf: 0 bis 2,5 m³/h
Abgas: 2,5 m³/h
Zulauf: 1,5 kg/h

Der Wassergehalt der resultierenden Reaktionsmischung betrug zwischen 29 und 30 %.

Die Analyse der organischen Bestandteile erfolgte wiederum mittels HPLC und einer Aminex-Säule. Es wurden 92 Gew.-% Propandiol-1,2 und 4,3 Gew.-% n-Propanol sowie geringe Mengen an niedrigen Alkoholen, aber kein Glycerin gefunden.

## Patentansprüche

1. Verfahren zur Herstellung von Propandiol-1,2 durch die katalytische Hydrierung von Glycerin bei erhöhter Temperatur und erhöhtem Druck, dadurch gekennzeichnet, daß man Glycerin mit einem Wassergehalt von bis zu 20 Gew.-% einsetzt und einen Katalysator verwendet, der die Metalle Cobalt, Kupfer, Mangan und Molybdän in Mengen von, bezogen auf die Gesamtmasse des Katalysators,
40 bis 70 Gew.-% Cobalt
10 bis 20 Gew.-% Kupfer
0 bis 10 Gew.-% Mangan und
0 bis 10 Gew.-% Molybdän
enthält, wobei dieser katalytisch aktiven Masse außerdem noch anorganische Polysäuren und/oder Heteropolysäuren in einer Menge von bis zu 10 Gew.-%, bezogen auf die Gesamtmasse des Katalysators, zugesetzt sein können.

## Claims

1. A process for the preparation of 1,2-propanediol by catalytic hydrogenation of glycerol at elevated temperature and pressure, which comprises using glycerol having a water content of up to 20% by weight and a catalyst comprising the metals cobalt, copper, manganese and molybdenum in amounts of, based on the total weight of the catalyst,
from 40 to 70% by weight of cobalt,
from 10 to 20% by weight of copper,
from 0 to 10% by weight of manganese and
from 0 to 10% by weight of molybdenum,
where this catalytically active material may additionally contain inorganic polyacids and/or heteropolyacids in an amount of up to 10% by weight, based on the total weight of the catalyst.

## Revendications

1. Procédé de préparation de propanediol-1,2 par hydrogénation catalytique de glycérol à température élevée et pression élevée, caractérisé en ce que l'on utilise du glycérol ayant une teneur en eau allant jusqu'à 20 % en poids, et un catalyseur contenant les métaux cobalt, cuivre, manganèse et molybdène en des quantités de
40-70 % en poids de cobalt,
10-20 % en poids de cuivre,
0-10 % en poids de manganèse et
0-10 % en poids de molybdène,
par rapport à la masse totale du catalyseur, cette masse active catalytiquement pouvant également contenir des polyacides et/ou des hétéropolyacides organiques en une quantité allant jusqu'à 10 % en poids, par rapport à la masse totale du catalyseur.
